# EUROPEAN PATENT SPECIFICATION

(11) **EP 0 594 607 B1**
(45) Date of publication and mention of the grant of the patent: **27.08.1997**
(21) Application number: 91906685.2
(22) Date of filing: 04.03.1991
(51) Int. Cl.: A61K 39/00, A61K 31/715, A61K 38/39, A61K 38/00

(54) **ENHANCEMENT OF THE DOWN-REGULATION OF AUTOIMMUNE DISEASES BY ORAL OR ENTERAL ADMINISTRATION OF AUTOANTIGENS**
VERSTÄRKUNG DER UNTERDRÜCKUNGSREGULATION VON AUTOIMMUNKRANKHEITEN DURCH ORALE ODER ENTERALE VERABREICHUNG VON AUTOANTIGENEN
AMELIORATION DE LA REGULATION REDUCTRICE DE MALADIES AUTOIMMUNES PAR ADMINISTRATION ORALE OU ENTERALE D'AUTOANTIGENES

(30) Priority: 02.03.1990 US 487732
(43) Date of publication of application: 04.05.1994
(60) Divisional application: 97100981.6
(73) Proprietor: AUTOIMMUNE, INC., Lexington, MA 02174 (US)
(72) Inventor: Weiner, Howard L.,, Brookline MA 02215 (US); Hafler, David Allen, West Newton MA 02165 (US); Khoury, Samia J., Brookline MA 02215 (US)
(74) Representative: Grünecker, Kinkeldey, Stockmair & Schwanhäusser Anwaltssozietät
(86) International application number: US9101466
(87) International publication number: WO9112816

(56) References cited:
- US-A- 4 695 459
- JOURNAL OF IMMUNOLOGY. vol. 127, no. 1, July 1981, BALTIMORE US pages 13 - 16 S. RAZIUDDIN ET AL. 'PREVENTION OF EXPERIMENTAL ALLERGIC ENCEPHALOMYELITIS BY BACTERIAL LIPOPOLYSACCHARIDES: INHIBITION OF CELL-MEDIATED IMMUNITY.'
- JOURNAL OF IMMUNOLOGY, vol. 127, no. 1, July 1981, BALTIMORE US; PP.16-20, S. RAZIUDDIN ET AL. 'EXPERIMENTAL ALLERGIC ENCEPHALOMYELITIS IN LEWIS RATS: INHIBITION BY BACTERIAL LIPOPOLYSACCHARIDES AND ACQUIRED RESISTANCE TO REINDUCTION BY CHALLENGE WITH MYELIN BASIC PROTEIN.'
- CHEMICAL ABSTRACTS, vol. 87, no. 25, 19 December 1977, Columbus, Ohio, US; abstract no. 199041p, K. NAGASAWA ET AL. 'PROTECTION AGAINST EXPERIMENTAL ALLERGIC ENCEPHALOMYELITIS IN GUINEA PIGS BY BACTERIAL LIPOPOLYSACCHARIDE.' page 531 ;
- CHEMICAL ABSTRACTS, vol. 91, no. 19, 5 November 1979, Columbus, Ohio, US; abstract no. 155887x, K. NAGASAWA ET AL. 'CORRELATION BETWEEN THE CAPACITY OF BACTERIAL LIPOPOLYSACCHARIDE TO SUPRESS EXPERIMENTAL ALLERGIC ENCEPHALOMYELITIS AND ITS MITOGENIC ACTIVITY FOR LYMPH NODE CELLS IN GUINEA PIGS.' page 488 ;
- PATENT ABSTRACTS OF JAPAN vol. 9, no. 309 (C-318)(2032) 5 December 1985
- IMMUNOLOGY vol. 58, no. 4, August 1986, OXFORD, GB pages 677 - 683 A. MOWAT ET AL. 'DIVERGENT EFFECTS OF BACTERIAL LIPOPOLYSACCHARIDE ON IMMUNITY TO ORALLY ADMINISTERED PROTEIN AND PARTICULATE ANTIGENS IN MICE.'
- CELLULAR IMMUNOLOGY vol. 131, no. 2, December 1990, NEW YORK, N. Y., US pp. 302 - 310 S.J. KHOURY ET AL. 'SUPPRESSION OF EXPERIMENTAL AUTOIMMUNE ENCEPHALOMYELITIS BY ORAL ADMINISTRATION OF MYELIN BASIC PROTEIN.'
- The Journal of Immunology, Vol. 140, No. 2, issued 01 January 1988, HIGGINS et al., "Suppression of Experimental Autoimmune Encephalomyelitis by Oral Administration of Myelin Basic Protein and its Fragments", pages 440-445, see entire article.
- Journal of Experimental Medicine, Vol. 169, issued May 1989, CLAYTON et al., "Peptide-specific Prevention of Experimental Allergic Encephalomyelitis", pages 1681-1691, see entire article.
- Journal of Immunology, Vol. 126, No. 4, issued April 1981, LANDO et al., "The Immunologic Response in Mice Unresponsive to Experimental Allergic Encephalomyelitis", pages 1526-1528, see Abstract.
- Science, Vol. 244, issued 19 May 1989, LOHSE et al., "Control of Experimental Autoimmune Encephalomyelitis by T cells Responding to Activated T cells", pages 820-822, see entire article.
- Nature, Vol. 341, issued 12 October 1989, VANDERBARK et al., "Immunization with a Synthetic T Cell Receptor V-region Peptide Protects Against Experimental Autoimmune Encephalomyelitis", pages 541-544, see entire article.
- Journal of Autoimmunity, Vol. 1, No. 5, issued October 1988, JONKER et al., "Successful Treatment of EAF in Rhesus Monkeys with MHC class II specific Monoclonal Antibodies", pages 399-414, see Abstract No. 89322538.

## Description

### BACKGROUND OF THE INVENTION

### Field of the Invention

The present invention relates to an improvement in the treatment of autoimmune diseases and in particular T-cell-mediated or T-cell-dependent autoimmune diseases. More specifically, the present invention is directed to the use of synergists to augment the activity of orally or enterally administered autoantigens, or disease-suppressive fragments or analogs thereof, in the prophylactic and therapeutic treatment of autoimmune diseases.

### Brief Description of the Background and Prior Art

Autoimmune diseases are characterized by an abnormal immune response (involving either cells or antibodies) directed against normal autologous tissues.

A number of strategies have been used or proposed to suppress autoimmune diseases, most notably drugs, such as clyclophosphamide, cyclosporin A, methotrexate, and Imuran (azothioprine). Steroid compounds, such as prednisone and methylprednisolone are also employed in many instances. These drugs have limited long term efficacy against both cell- and antibody-mediated autoimmune diseases. Use of such drugs is limited by virtue of their toxic side effects to other organ systems and also because they induce "global" immunosuppression in a patient receiving prolonged treatment with these drugs, e.g. the normal protective immune response to pathogenic microorganisms is downregulated thereby increasing the risk of infections caused by these pathogens. A further drawback is that there is an increased risk that malignancies will develop in patients receiving prolonged global immunosuppression.

Other therapies have been proposed for the treatment of autoimmune diseases. International Patent Application WO 88/10120 discloses that the oral or enteral administration of myelin basic protein (MBP) and disease-inducing and non-inducing fragments and analogs thereof is effective in suppressing acute monophasic experimental allergic encephalomyelitis (EAE), an induced T-cell mediated autoimmune disease directed against myelin basic protein (MBP), see also J. Immunology, vol.140, p.440-445, (1988). EAE is a recognized and widely used animal model for the human disease multiple sclerosis (MS). The above-identified applications also disclose that the oral or enteral administration of Mycobacterium tuberculosis is an effective treatment for suppressing adjuvant arthritis and extrapolate the aforementioned results to the treatment of other autoimmune diseases.

International Patent Application WO 91/01333 discloses the oral or enteral administration of S-antigen for the treatment of autoimmune uveoretinitis.

International Patent Application WO 91/08760 published after the filing date of the present application discloses the aerosol administration of autoantigens for the treatment of cell-and antibody-mediated autoimmune diseases.

Nagler-Anderson et al. (Proc. Natl. Acad. Sci. (USA) 83: 7443-7446, 1986) describe the oral administration of collagen to suppress collagen-induced arthritis in a mouse model.

Various methods have been employed to induce antigen-specific suppression of EAE such as immunization with MBP emulsified in incomplete Freund's adjuvant, (Lando, Z. et al., J. Immunol. 126: 1526 (1981)), and intravenous injection of MBP-conjugated lymphoid cells (Sriram, S. et al., Cell. Immunol. 75: 378 (1983)).

Traugott et al., J. Neurol. Sci. 56:65-73 (1982), and Raine et al., Lab. Investigation 48: 275-84 (1983) disclose that treatment of a strain of guinea pigs suffering from chronic relapsing EAE by parenteral administration of MBP alone or in incomplete Freund's adjuvant (IFA) or in combination with galactocerebroside, a lipid hapten of myelin, suppressed the clinical EAE symptoms.

McKenna et al., Cell. Immun. 81: 391-402 (1983), disclose that preinjection of rats with guinea pig MBP coupled to syngeneic spleen leukocytes or to syngeneic red blood cells suppressed (in a dose-dependent manner) the subsequent induction of EAE using guinea pig MBP in Freund's complete adjuvant.

Based on an English language abstract, Belik et al., Vopr. Med. Khim. 24: 372-377 (1978), disclose (according to an English language abstract) parenteral administration of "alkaline myelin protein fragment" (AMPF) and "synthetic encephalitogenic peptide" (SEP) to guinea pigs with EAE. The animals had been sensitized by bovine AMPF or by SEP but recovered after AMPF administration.

Braley-Mullen et al., Cell. Immun. 51: 408 (1980), and Nagler-Anderson et al. noted above, both disclose the suppression of the symptoms of two other experimental autoimmune diseases which are induced by injection of animals with autoantigen-lymphocyte conjugates. Braley-Mullen et al. reports the suppression of experimental autoimmune thyroiditis in the guinea pig by injection of thyroglobulin antigen in IFA. Nagler-Anderson et al. reports the suppression of Type II collagen-induced arthritis in the mouse by intragastric administration of soluble, but not denatured, Type II collagens prior to immunization of the animal with Type II collagen in adjuvant.

Newby, T.J. et al. (Local Immune Responses in the Gut, p. 1191, CRC Press, Boca Raton, FL, 1984; Immunol. 41: 617-621, 1980) report that feeding bacterial LPS to mice can enhance tolerance to contact-hypersensitivity induced by orally administered antigen (picryl chloride). However, simultaneous oral administration of LPS and picryl chloride had no statistically significant effect in delayed-type hypersensitivity (DTH) reactions to this antigen.

Michalek et al. (J. Immunol. 128: 1992-1998, 1982) report that prolonged gastric intubation (i.e. enteral administration) of sheep red blood cells (SRBC) followed by systemic administration of SRBC resulted in enhanced splenic immune responses in LPS-unresponsive mice and oral tolerance in LPS-responsive mice. There was no coadministration of LPS.

Mowat, A.M. et al. Immunol. 58: 677-683, 1986) disclose that simultaneous administration of LPS (intravenously) to normal mice fed ovalbumin prevented the induction of oral tolerance but did not alter the Delayed-type hypersensitivity (DTH) responses to fed ovalbumin.

Hamada, T. et al. (Autoimmunity 2: 275-284, 1989) report that LPS treatment in vitro of MBP-sensitized lymphoid cells augmented the adoptive transfer of EAE in Lewis Rats.

Due to the progressive, debilitating nature of autoimmune diseases, what is needed in the art are improved methods for treating autoimmune diseases.

It is an object of the present invention to provide pharmaceutical formulations for treating mammals suffering from autoimmune diseases.

### SUMMARY OF THE INVENTION

The present invention is directed to an improved pharmaceutical formulation for treating or preventing a T-cell mediated or T-cell dependent autoimmune disease (AD) which comprises:
(a) an AD-suppressive agent in an oral or enteral dosage form selected from the group consisting of (i) autoantigens specific for said disease; (ii) AD-suppressive fragments of said autoantigens; and (iii) AD-suppressive analogs of said autoantigens or said fragments; and
(b) at least one compound having the property of enhancing the AD-suppressive activity of said agent wherein said compound administered in combination with said AD-suppressive agent imports to said formulation one or both of the following properties:
   (i) the property of inducing production and/or secretion of interleukin-1; and (ii) the property of decreasing expression of class II histocompatibility molecules by gut epithelial cells;
and (c) a physiologically accentable carrier or diluent.

The amount of the enhancing compound should be effective to potentiate the AD-suppressive agent and effective to enhance the suppressive activity of the AD-suppressant if it is administered at a level already effective to suppress AD symptoms.

The activity-enhancing compound (which will be often referred to herein as a "synergist") need not be administered orally or enterally; however, oral or enteral administration is preferred. Furthermore, any order of administration of the suppressive agent and the synergist may be used although substantially simultaneous administration is preferred.

### BRIEF DESCRIPTION OF THE FIGURES

Figure 1 is a bar graph showing the effect of feeding MBP-LPS on delayed-type hypersensitivity (DTH) reactions in rats.

Figure 2 is a bar graph showing the effect of feeding MBP-Lipid A or DTH responses in rats.

Figure 3 (A-C) are a series of bar graphs showing the effect of feeding MBP + LPS on chronic EAE in guinea pigs.

### DESCRIPTION OF THE PREFERRED EMBODIMENTS

The treatment of T-cell-mediated or T-cell-dependent autoimmune disease by (a) the oral or enteral administration of an AD-suppressive agent and (b) administration of one or more compounds that enhance the AD-suppressive activity of the agent "treatment" is meant to include both prophylactic treatment to prevent autoimmune diseases (or the manifestation of clinical symptoms thereof) as well as the therapeutic suppression or alleviation of symptoms after the onset of such autoimmune diseases.

The term "mammal" covers all life forms that have an immunoregulatory system and are therefore susceptible to autoimmune diseases.

An autoimmune disease is a malfunction of the immune system of mammals, including humans, in which the immune system fails to distinguish between foreign substances within the mammal and autologous tissues or substances and, as a result, treats autologous tissues and substances as if they were foreign.

An "autoantigen" is any substance normally found within a mammal that, in an abnormal situation, is no longer recognized as part of the mammal itself by the lymphocytes or antibodies of that mammal, and is therefore attacked by the immunoregulatory system as though it were a foreign substance. The term also includes antigenic substances which induce conditions having the symptoms of an autoimmune disease when administered to mammals.

The term "AD-suppressive fragment(s)" of such autoantigens includes any peptide or polypeptide or other compound comprising partial amino acid sequences or moieties of autoantigens and possessing the ability to suppress or prevent T-cell-mediated or T-cell-dependent autoimmune response, upon oral or enteral administration. It should be noted that such fragment need not also possess the autoantigenic properties of the entire autoantigen. For example, with respect to MBP, which when administered parenterally with an adjuvant induces EAE in susceptible mammals, it was discovered that certain non-disease-inducing fragments of MBP nevertheless possessed AD-suppressive activity as disclosed in International Patent Application WO 88/10120

The term "analog(s)" of such autoantigens or fragments includes compounds that are so structurally related to these autoantigens or to their AD-suppressive fragments that they possess the same biological activity, i.e., the ability to eliminate or suppress T-cell-mediated or T-cell-dependent autoimmune response, upon oral or enteral administration. For example, the term includes peptides having amino acid sequences which differ from the amino acid sequence of the autoantigen by one or more amino acid residues (while still retaining the AD-suppressive activity) as well as compounds or compositions which mimic the AD-suppressive activity of the autoantigens in their ability to suppress or alleviate the symptoms of the disease. An example of such compositions is tissue from an organ that is the target of attack in an autoimmune disease.

"Autoimmune-disease suppressive agent" or "AD-suppressive agent" is a compound or composition which when administered orally or enterally to a mammal suppresses, prevents or delays the clinical manifestation of a particular autoimmune disease. The term includes autoantigens specific for an autoimmune disease, as well as AD-suppressive fragments or analogs thereof as defined above.

"Synergists" are defined herein as substances which augment or enhance the suppression of the clinical manifestation of autoimmune diseases when administered orally in conjunction with administration of AD-suppressive agents. As used in the preceding sentence "in conjunction with" (also referred to herein as "in association with") means before, substantially simultaneously with or after oral or enteral administration of the AD-suppressive agent. Naturally, administration of the synergist should not precede nor follow that of the AD-suppressive agent by so long an interval of time that the effects of the substance administered first have worn off. Therefore, the synergists should be administered within 24 hours of the AD-suppressive agent.

Examples of synergists for use in the present invention include, but are not limited to bacterial lipopolysaccharides from a wide variety of gram-negative bacteria such as various subtypes of E, coli or Salmonella (LPS, Sigma Chemical Co. St. Louis, MO, Difco, Detroit, MI BIOMOL Res. Labs, Plymouth Meeting, PA), Lipid A (Sigma Chemical Co., ICN Biochemicals, Cleveland, OH, Polysciences Inc., Warrington, PA), and immunoregulatory lipoproteins, such as peptides covalently linked to tripalmitoyl-S-glycarylcysteinyl-seryl-serine (P₃CSS), which can be obtained as disclosed in Deres, K. et al. Nature 342: 561-564, 1989) or "Braun's", lipoprotein from E. coli which can be obtained as disclosed in Braun, V., Biochim. Biophys. Acta 435: 335-337, 1976. LPS is preferred and Lipid A is particularly preferred. Lipid A is particularly preferred for use in the present invention because it is less toxic than the entire LPS molecule. LPS for use in the present invention can also be extracted from gram-negative bacteria and purified using the methods of Galanes, C. et al. (Eur J. Biochem. 9: 245, 1969) and Skelly, R. R. et al. (Infect. Immun. 23: 287, 1979).

One common feature of the biologically active materials which can be used as synergists in the present invention is that they induce the production and/or secretion of interleukin-1 (IL-1).

IL-1 is a protein produced by many normal and transformed cells, and is predominantly produced by macrophages, monocytes and keritanocytes. Microbial products, such as endotoxins, exotoxins, yeast cell walls, and viral hemagglutinins induce IL-1 production by monocytes. IL-1 has a broad spectrum of biological activities on cells of the immune system, as well as cells outside the immune system, and is considered to be a major inflammatory mediator.

Administration of synergists in association with oral or enteral administration of an AD-suppressive agent markedly enhances the activity of the agent in suppressing the symptoms of the autoimmune disease for which the agent is specific. In other words, the efficacy of treatment of the target autoimmune disease is increased by co-administration of the synergist (compared with the efficacy of using the AD-suppressant agent alone).

According to the present invention, after an AD-suppressive agent is administered orally or enterally (i.e. directly into the stomach) it passes into the small intestine where it comes in contact with Peyers Patches cells which are collections of immunocytes located under the intestinal wall. These cells as well as intraepithelial lymphocytes (located under the intestinal epithelium) are in communication with the immune system including the spleen and lymph nodes. The result is that tolerance to the AD-suppressive agent is induced and the immune response directed against the mammal's own tissues is attenuated or entirely suppressed. The thus-induced tolerance is specific for the autoimmune disease: no effect has been discerned on the ability of the treated mammal to mount a response against a pathogen. Moreover, oral or enteral administration of an irrelevant antigen (i.e. one not implicated in an autoimmune disease, such as bovine serum albumin) produced no effect on the clinical manifestation of the autoimmune disease or on the susceptibility of a mammal to an inducible condition (such as EAE or AA) serving as a model for an autoimmune disease.

The tolerance induced by the AD-suppressive agents is dose-dependent: over a broad oral or enteral dosage range, the suppression (or attenuation) of clinical and histological manifestations of the disease increases with increasing dosage of AD-suppressive agent ingested.

The ability of the synergist to enhance orally-induced tolerance by an autoantigen is also dose-dependent, i.e. it increases with increasing dose of synergist. But because the enhancement by the synergist of orally induced tolerance is indeed synergistic, use of the synergist is anticipated to continue to improve tolerance induced by relatively high oral or enteral doses of the AD-suppressive agents. Conversely, associated use of the synergist is anticipated to permit lower doses of AD-suppressive agent to be used for the same level of clinical symptom suppression to be achieved.

The activity of the synergist in enhancing orally-induced tolerance is not specific to the particular AD-suppressive agent. Both EAE and DTH responses were down-regulated upon co-administration of the specific autoantigen and the synergists. Thus, the ability of the synergist to enhance orally-induced specific tolerance to an autoantigen can be extrapolated to any other AD-suppressive agent encompassed by the present invention. Yet, administration of the synergist alone has produced minimal or no effect in attenuating either the specific immune response of a mammal to a particular autoantigen, or the ability of the mammal to mount an immune response to an exoantigen (i.e., a true foreign antigenic substance). Use of the synergist thus affords this important two-fold advantage among others: enhancement of the suppression of the specific immune response to an autoantigen and no substantial effect on the ability of the subject so treated to mount an immune response against invading pathogens.

Various model systems have been developed for studying autoimmune diseases. Experimental allergic encephalomyelitis (EAE) has been studied in mice and other mammalian species as a model for Multiple Sclerosis (MS). The disease is induced by parenteral administration of MBP and an adjuvant (such as Freund's complete adjuvant). This treatment induces both a monophasic and an exacerbating/remitting form of demyelinating disease (depending on the species and details of administration). The induced disease has the characteristics of MS. Parenteral administration of Mycobacterium tuberculosis with Freund's complete adjuvant oil into the dorsal root tail of susceptible mammals induces a disease with the characteristics of human rheumatoid arthritis. In addition, the administration to Lewis rats of S-antigen and an adjuvant induces autoimmune uveoretinitis whereas diabetes develops spontaneously in the NOD mouse and the BB rat. Various ones of these model systems may be employed to demonstrate the efficacy and improved treatment provided by the present invention.

Non-limiting examples of autoimmune diseases which are cell-mediated include multiple sclerosis, rheumatoid arthritis, autoimmune uveoretinitis, diabetes (especially Juvenile Onset diabetes) and autoimmune thyroiditis. A non-limiting list of disease models and the specific autoantigens effective in the treatment of these diseases when administered in an oral or enteral form are set forth in Table 1 below.

**TABLE 1**

| Disease Model | Specific Autoantigen |
|---|---|
| Multiple Sclerosis | MBP |
| Rheumatoid Arthritis | Collagen |
| Autoimmune Thyroiditis | Thyroglobulin |
| Autoimmune uveoretinitis | S-antigen |
| Diabetes | Islet cell extract |
| Chronic Active Hepatitis | Liver extract |
| Adrenalitis | Adrenal gland extract |
| Polymyositis | Muscle extract |

For any autoimmune disease, tissue extracts can be used as well as the specific antigens described above.

Other autoimmune diseases and their specific autoantigens and/or target tissues are disclosed in Schwartz, R.S. et al. in Fundamental Immunology, Second Edition, Paul W.E., Ed., pg 819-859, Raven Press, NY, 1989.

Autoantigens for use in the present invention can be isolated from the tissue which is the target for the particular autoimmune disease. For example, myelin basic protein (MBP) for use in treating MS can be isolated and purified from mammals using the method of Diebler et al. (infra) as shown in Example 1 below.

When treating a disease having the symptoms of rheumatoid arthritis, collagen can be isolated and purified by the method of Trentham et al., J. Exp. Med. 146: 857, 1977.

For treating autoimmune uveoretinitis, purified S-antigen can be obtained as described in copending Int. Patent Application WO 91/01333.

Fragments and analogs of autoantigens for use in the present invention can be synthesized using solid phase synthesis techniques well-known in the art such as those of Merrifield, R.B. (Fed. Proc. Am. Soc. Ex. Biol. 21: 412, 1962 and J. Am. Chem. Soc. 85: 2149, 1963) and Mitchel, A.R. et al., (J. Am. Chem. Soc. 98: 7357, 1976). as well as Tam, J. et al., (J. Am. Chem. Soc. 105: 6442, 1983) Analogs can be constructed by identifying an equivalent amino acid sequence and using the peptide synthesis techniques disclosed above.

Analogs can be provided using for example the known amino acid sequence of GP-MBP as disclosed in G. Hashim, in Myelin: Chemistry and Biology Alan R. lisa, N.Y., 1980 using techniques described above and in Eyler, E.H., in Advances in Experimental Medicine and Biology 98: 259-281, 1978. For example, a peptide having a sequence corresponding to guinea pig MBP (GP-MBP) amino acid residues 72-85 as disclosed in Hashim (supra) can be chemically synthesized using the above-described techniques with an amino acid substitution at the terminal asparagine position to glutamine. The peptide can be tested for disease-suppressive activity when administered in oral or enteral form using the techniques described in Example 2 below.

Disease-suppressive analogs and fragments can also be obtained using recombinant DNA techniques well-known in the art.

The present invention provides pharmaceutical formulations for treating mammals suffering from autoimmune diseases comprising an amount of a synergist (as described below) effective to augment the autoimmune disease-suppressive effect of the AD-suppressive agent, a pharmaceutically acceptable carrier or diluent and an AD-suppressive agent specific for the autoimmune disease in an amount effective (singly or in conjunction with the amount of the synergist) to treat or prevent the clinical symptoms of specific autoimmune disease.

Each formulation according to the present invention may additionally comprise inert constituents including pharmaceutically acceptable carriers, diluents, fillers solubilizing or emulsifying agents, and salts as is well-known in the art. For example, tablets may be formulated in accordance with conventional procedures employing solid carriers well-known in the art. Capsules employed in the present invention may be made from any pharmaceutically acceptable material such as gelatin or cellulose derivatives. Sustained release oral delivery systems and/or enteric coatings for orally administered dosage forms are also contemplated such as those described in U.S. Patent No. 4,704,295 issued November 3, 1987, U.S. Patent No. 4,556,552 issued December 3, 1985, U.S. Patent No. 4,309,404 issued January 5, 1982, and U.S. Patent No. 4,309,406 issued January 5, 1982.

Examples of solid carriers include starch, sugar, bentonite, silica and other commonly used carriers. Further non-limiting examples of carriers and diluents which may be used in the formulations of the present invention include saline, syrup, dextrose and water.

It will be appreciated that the unit content of active ingredient or ingredients contained in an individual dose of each dosage form need not in itself constitute an effective amount since the necessary effective amount can be reached by administration of a plurality of dosage units (such as capsules or tablets or combinations thereof).

The route of administration of the synergists is preferably oral or enteral. Preferred oral or enteral pharmaceutical formulations may comprise for example, a pill or capsule containing between about 1 mg and about 300 mg of one or more synergists of the present invention with or without an effective amount of the AD-suppressive agent specific for the autoimmune disease.

In general, the autoantigen, fragment, or analog is introduced to a mammal, orally or enterally, in an amount of between about 15 micrograms per kg body weight of said mammal and about 15 mg per kg body weight of said mammal per day, and may be administered in single dose form or multiple dose form. Preferably the autoantigen, fragment, or analog is administered in an amount between about 300 micrograms and about 12 mg per kg body weight of said mammal per day.

The synergist may be administered to a mammal preferably orally or enterally in an amount broadly ranging between about 15 mg and about 15 mg per kg body weight of said mammal per day and preferably ranging between about 300 mg and about 12 mg per kg body weight per day and also may be administered in a single or multiple dose form. The synergist may be orally or enterally administered before (within 24 hours and preferably within 1 hour) or substantially simultaneously with or after (within 24 hours after) the administration of the autoantigen or biologically active fragment or analog of said autoantigen specific for said disease. As will be understood by one skilled in the art, the exact dosage and frequency of administration is a function of the activity of the AD-suppressive agent and the synergist as well as the age, sex, weight, and physical condition of the subject to be treated, and the concurrent administration or absence of other treatments. Consequently, adjustment of the dosages used and administration schedules must be determined based on these factors and may need to be determined experimentally. Such determination, however, requires no more than routine experimentation, as disclosed by Higgins and Weiner for EAE in J. Immunol 140: 440-445, 1988.

As shown in Example 8 below, subcutaneous administration of IL-1 enhanced the AD-suppressive effect of orally administered MBP on EAE. This is a most surprising finding in that IL-1 has been found to have an immunostimulatory effect on helper T-cells, the mediators of EAE and thus would be expected to exacerbaxe disease symptoms. Therefore, although oral or enteral administration of the synergist (which induces IL-1 production and/or secretion) is preferred, parenteral administration of IL-1, and preferably subcutaneously, to a mammal at a concentration ranging between about 0.2 mg and about 60 mg per kg of body weight of said mammals, combined with oral or enteral administration of an AD-suppressive agent (in dosages described above) is also within the scope of the present invention. IL-1 is commercially available from numerous commercial sources such as Boehringer Mannheim (Indianopolis, IN), Amgen Biologicals (Thousand Oaks, CA) and ICN Biochemicals (Cleveland, OH).

Where the AD-suppressive agent and synergist are introduced orally, they may be mixed with other food forms and consumed in solid, semi-solid, suspension, or emulsion form; they also may be mixed with pharmaceutically acceptable carriers, flavor enhancers and the like.

Where the AD-suppressive agent and synergist are administered enterally, they may be introduced in solid, semi-solid, suspension or emulsion form and may be compounded with any of a host of well-known, pharmaceutically acceptable carriers, including water, suspending agents, and emulsifying agents.

The AD-suppressive agent and the synergist may be administered together in the same dosage form, co-administered by ingesting separate dosage forms, or administered sequentially in separate dosage forms.

Examples 2-8 illustrate the use of a synergist in conjunction with an AD-suppressive agent and the specific tolerance-enhancing effect of such joint use.

According to Examples 2-8, feeding bacterial lipopolysaccharide (LPS) or the Lipid A moiety of LPS (LPS is composed of a Lipid A moiety and a polysaccharide moiety) used as the synergist enhanced the protective effect of feeding MBP on EAE in Lewis rats. The induction of disease, the disease severity and the Disease Index were all reduced as a result of administration of an AD-suppressive agent with a synergist as opposed to the oral administration of an AD-suppressive agent alone. The synergists were also effective in suppressing disease symptoms when administered with the autoantigen during the active disease phase as shown in Example 6 using a chronic EAE model in guinea pigs.

The feeding of MBP-plus-LPS or MBP-plus-Lipid A also led to a significant decrease in the delayed type hypersensitivity (DTH) reactions of the treated animals. EAE and DTH are both believed to be CD4+ T-cell-mediated phenomena. Consequently, the decrease in DTH corroborates the effect of the synergists. In both the EAE and DTH systems Lipid A or LPS had little or no effect when added alone in the absence of the autoantigen, MBP. Therefore, the tolerance enhancing effect of the synergist is not nonspecific.

It is anticipated that the synergists will augment or enhance the suppressive effect obtained by the oral or enteral administration of AD-suppressive agents in the treatment of other autoimmune diseases such as arthritis, diabetes, as well as the autoimmune diseases such as those exemplified above in Table 1.

In addition, as shown in Example 7 below, orally-administered MBP plus LPS caused a decreased expression of Class II histocompatibility molecules relative to Class I histocompatibility molecules in MBP-fed rats on gut epithelial cells isolated from treated animals. Without wishing to be bound by theory, it is hypothesized that by differentially decreasing Class II-expression, LPS may cause a reduction in antigen presentation to CD4+ T-cells (helper T-cells), which are inducer cells. Since oral MBP tolerance induction is believed to operate via a different mechanism, i.e. induction of suppressor T-cells, a synergistic effect is observed when LPS and MBP are administered together.

The present invention is further described below in specific Examples.

### EXAMPLE 1:

Autoantigens used in practicing the method of the present invention were obtained using the method and techniques set forth below.

GP-MBP was purified by the method of Diebler, G.E. et al. (Prep. Biochem. 2: 139, 1972) from guinea pig brain tissue and was obtained from Pel Freeze (Rogers, Arkansas). Briefly, central nervous system tissue was isolated and homogenized in a chloroform-methanol solution, extracted in acetone, filtered and resuspended in the same solution. The solution was extracted in acetone, filtered, resuspended in water, adjusted to pH 3.0 and incubated for 1 hour. The solution was then centrifuged and extracted with 8 M urea, CM-5 added, the pH adjusted to 11, and the solution filtered and resuspended twice in urea. The solution was then filtered again, resuspended twice in water, filtered, resuspended in 0.121 N HCl, filtered, dialyzed against 10 volumes of distilled water and lyophilized before use.

### EXAMPLE 2: Feeding LPS Enhances the Protective Effect of Feeding MBP on EAE

A series of experiments were performed in the Lewis rat in which Lewis rats age 6-8 weeks (Harlan-Sprague Dawley, Indianopolis, IN) were fed MBP (1 mg in PBS) or LPS (1 mg in PBS) alone or a combination of MBP + LPS (1 mg and 1 mg) by gastric intubation using an 18-gauge stainless steel animal feeding needle (Thomas Scientific, Swedesboro, NJ). The rats were fed five times (total dose 5 mg) before immunization. LPS (Sigma Chemical Co. St. Louis, MO) was from E. coli strain 0127:B8. The rats were then immunized in each foot pad with 25 micrograms MBP in an equal volume of complete Freund's adjuvant (CFA) containing 4 micrograms of Mycobacterium tuberculosis (Difco, Detroit, MI) 2 days after the last feeding and observed for EAE disease over the following 2-3 weeks. Disease is manifested by paralysis. The seventy scoring system was: 0 = normal; 1 = limp tail; 2 = hind leg paralysis; 3 = incontinence; 4 = tetraplegia; and 5 = moribund. EAE severity varied between 2 and 5 depending upon the experiment. Disease severity was also scored as "Disease Index". The Disease Index was calculated for each animal and consisted of duration of disease (in days) times the highest disease score. Animals were scored clinically in a blinded fashion. The results are set forth in Table 2 below:

**TABLE 2**

| Oral LPS Enhances Orally Induced Suppression of EAE by MBP | | | | |
|---|---|---|---|---|
| **TREATMENT** | **INCIDENCE** | **DURATION (Days)** | **MEAN MAXIMAL SEVERITY** | **DISEASE INDEX** |
| None (Control) | 22/22 | 6.85 ± 0.39 | 2.98 ± 0.38 | 18.60 ± 2.03 |
| LPS fed | 20/24 | 4.58 ± 0.56^{b} | 1.89 ± 0.25^{a} | 11.15 ± 1.84^{a} |
| MBP fed | 21/29^{b} | 3.24 ± 0.50^{c} | 1.17 ± 0.18^{c} | 5.83 ± 1.18^{c} |
| MBP+LPS fed | 12/29^{b} (p<0.05)^{d} | 1.97 ± 0.53^{c} (N.S.)^{d} | 0.53 ± 0.13^{c} (p<0.05)^{d} | 2.72 ± 0.79^{c} (p<0.05)^{a} |

| | | | | |
|---|---|---|---|---|
| a p < 0.06 vs. control | | | | |
| b p < 0.01 vs. control | | | | |
| c p < 0.001 vs. control | | | | |
| d p values comparing LPS + MBP vs. MBP | | | | |
| [p values were calculated by student t-test except for the incidence, where chi-square was used.] | | | | |

The experiments described above demonstrated a marked augmentation of protection against the disease in animals that were fed both LPS and MBP as compared to those fed MBP alone. MBP + LPS lowered the incidence of disease (12/29 animals versus 22/22 for controls and 12/29 animals for MBP-fed), the duration of disease, the mean maximal severity and the DI. Of note is that feeding LPS alone had a lesser effect on the animals' susceptibility to EAE than either MBP or MBP + LPS. The results clearly demonstrate a superadditive or synergistic enhancement of disease suppression due to the joint use of an AD-suppressive agent (MBP) and a synergist (LPS).

### EXAMPLE 3: The Lipid A Moiety of LPS is Equally Effective

LPS is a structure that contains both a lipid moiety plus a polysaccharide chain (Chiller, J. M. et al., Proc. Nat. Acad. Sci USA 70: 2129-2133, 1973). In experiments performed it was found that Lipid A functioned equally well or better than LPS as a synergist in protecting against EAE. Rats (9-10 per group) were fed with Lipid A (75 micrograms per rat, from Salmonella minnesota Re 595, obtained from Sigma Chemical Co.), following the same protocol used for LPS feeding in Example 2 above. EAE was induced as described in Example 2 above. The results are set forth in Table 3 below.

**TABLE 3**

| Lipid A Is The Active Part of the LPS Molecule | | | | |
|---|---|---|---|---|
| **Treatment** | **Incidence** | **Duration (Days)** | **Maximal Grade** | **Disease Index** |
| None (Control) | 10/10 | 7.70 ± 0.26 | 3.1 ± 0.10 | 26.20 ± 1.94 |
| Lipid A | 9/9 | 7.56 ± 0.34^{a} | 3.40 ± 0.24^{a} | 24.33 ± 1.76^{a} |
| MBP | 10/10 | 6.40 ± 0.45^{b} | 1.9 ± 0.23^{c} | 11.79 ± 1.94^{d} |
| MBP+Lipid A | 3/9 | 3.44 ± 1.16^{c} (p < 0.05)^{e} | 0.03 ± 0.29^{d} (p < 0.01)^{e} | 6.50 ± 2.1^{d} (p < 0.05)^{e} |

| | | | | |
|---|---|---|---|---|
| a p not significant | | | | |
| b p < 0.06 | | | | |
| c p < 0.01 | | | | |
| d p < 0.001 | | | | |
| e p values of MBP vs. MBP+Lipid A | | | | |

As can be seen from the results presented in Table 3 above, Lipid A, at a dose of 75 micrograms administered orally with MBP, significantly enhanced protection against EAE with a decrease in the Disease Index (DI) from 12.12 for controls and 11.6 for MBP fed rats to 0.06 for rats fed MBP+Lipid A (Table 3). In addition, MBP+Lipid A fed rats had a lower incidence of disease (33% versus 100% for all other groups), a shorter duration of disease and a lower maximal grade when compared to control, MBP-fed or Lipid A-fed animals.

### EXAMPLE 4: Delayed Type Hypersensitivity Reactions

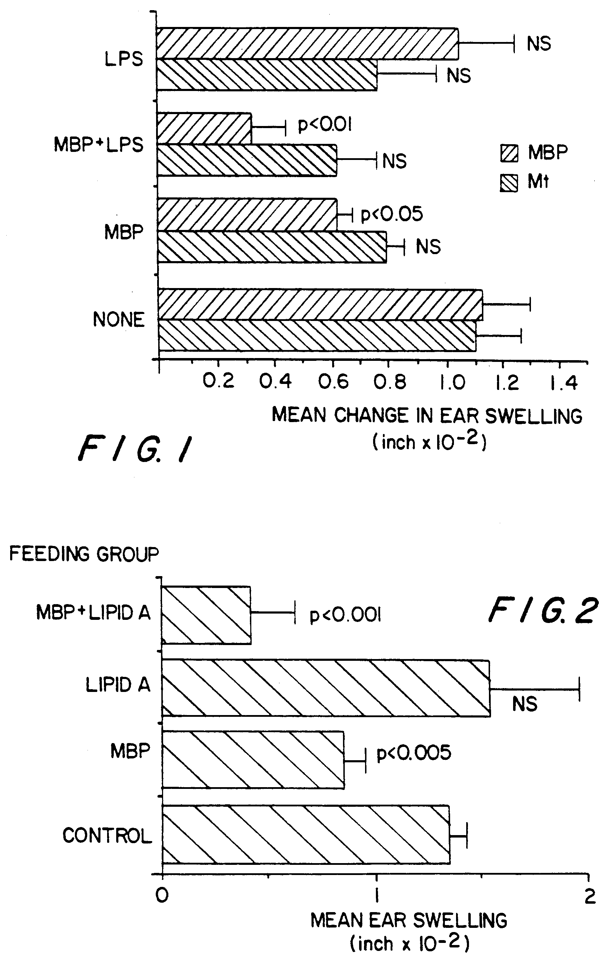

The experiments of Examples 2 and 3 outlined above monitor disease expression measured by paralysis. A series of experiments were performed to determine whether other immune measurements would also demonstrate a superadditive effect as a result of use of the synergist. Since EAE is believed to be a CD4+ T-cell mediated disease, and this cell is the primary cell involved in Delayed Type Hypersensitivity (DTH) reactions, DTH responses in animals treated with LPS were measured to determine whether suppression could be observed. These DTH reactions were done in a standard fashion by measuring ear swelling 48 hours following the injection of 50 microliters of the autoantigen (MBP, 200 micrograms/ml) into the pinna of a rats ear that were not exposed to MBP, sensitized with MBP 200 micrograms/ml, and/or fed 1 mg MBP and/or LPS (1 mg) prior to challenge. As a control, 50 microliters of Mycobacterium tubercolosis (Mt) at a concentration of 200 micrograms/ml was administered to the MBP sensitized rats. The measurements were performed as in J. Immunol. 125:483,1980 and are expressed as the mean change in ear swelling in inches x 10⁻². The results are shown in Figures 1 and 2. In Figures 1 and 2, the ordinate depicts the mean difference in thickness of the rats' ears before and 48 hours after MBP injection. Figure 1 depicts the DTH results in rats fed LPS and Figure 2 depicts the DTH results in rats fed Lipid A.

A marked and significant decrease (45%) in DTH responses was found in animals fed a combination of either MBP and Lipid A or LPS. Neither LPS nor Lipid A alone had any effect in inhibiting ear-swelling (Figures 1 and 2, respectively). The suppression of DTH following MBP and LPS or Lipid A adminstration was antigen specific, as DTH responses to Mt were unaffected.

### EXAMPLE 5: Route of Administration Influences Effect of Synergist

In order to study the mechanism of action of the synergists of the present invention, an experiment was performed according to the protocol as set forth in Example 2 above in which the synergist (LPS) was administered (at a dose of 1 milligram) at the same time as 1 milligram of MBP administered 5 times as in Example 2 above either orally ("PO") or subcutaneously ("SQ"). Controls were simply challenged with injected MBP. The results are set forth in Table 4 below.

**TABLE 4**

| LPS Enhances Oral Tolerance Better When Given Orally Than When Given By Another Route | | | | |
|---|---|---|---|---|
| **TREATMENT** | **INCIDENCE** | **DURATION** | **MEAN MAXIMAL SEVERITY** | **DISEASE INDEX** |
| None (Control) | 5/5 | 7.6 ± 0.51 | 3.2 ± 0.37 | 24.8 ± 3.78 |
| MBP fed | 5/5 | 5.4 ± 0.40^{a} | 1.8 ± 0.37^{b} | 10.0 ± 2.45^{b} |
| MBP+LPS PO | 2/5 | 1.8 ± 0.11^{b} (p<0.05)^{d} | 0.8 ± 0.49^{b} (p<0.05)^{d} | 3.6 ± 2.23^{a} (p<0.05)^{d} |
| MBP+LPS SQ | 4/4 | 6.0 ± 0.71^{c} | 2.7 ± 0.25^{c} | 15.52 ± 3.28^{c} |
| LPS SQ | 4/4 | 5.0 ± 0.47^{c} | 4.75 ± 0.25^{b} | 22.75 ± 5.4^{c} |

| | | | | |
|---|---|---|---|---|
| a p < 0.01 | | | | |
| b p < 0.05 | | | | |
| c NS | | | | |
| d p values of MEP+LPS PO vs. MEP+LPS SQ | | | | |

Oral LPS decreased the DI from 23.04 in controls and 8.0 in MBP fed rats to 0.26 in MBP+LPS fed rats. Subcutaneous LPS, on the other hand, abrogated the tolerance following MBP feeding, the DI was 16.5 compared to 8.0 for MBP alone (Table 4). DTH responses to MBP were measured in these rats, and the results correlate with those obtained for clinical disease. MBP with oral LPS suppressed the DTH response more than MBP alone (data not shown). In the rats treated with subcutaneous injection of LPS and oral MBP, mean ear swelling was not significantly different from those of control rats (data not shown).

### EXAMPLE 6: Administering the Synergist Post-Induction of EAE

In order to examine the effects of the synergists of the present invention on an active, ongoing autoimmune disease, a chronic EAE model was studied. Strain 13 guinea pigs (Associated Rabbit Industries, East Bridgewater, MA) were sensitized with a 1:1 mixture of guinea pig myelin (purchased from Pel-Freez, Rogers, AR) and sterile physiological saline with an equal volume of Incomplete Fruend's adjuvant containing 4 micrograms of heat killed Mycobacterium tuberculosis (Difco, Detroit, MI). 0.4 ml was injected into several sites in the nuchal area.

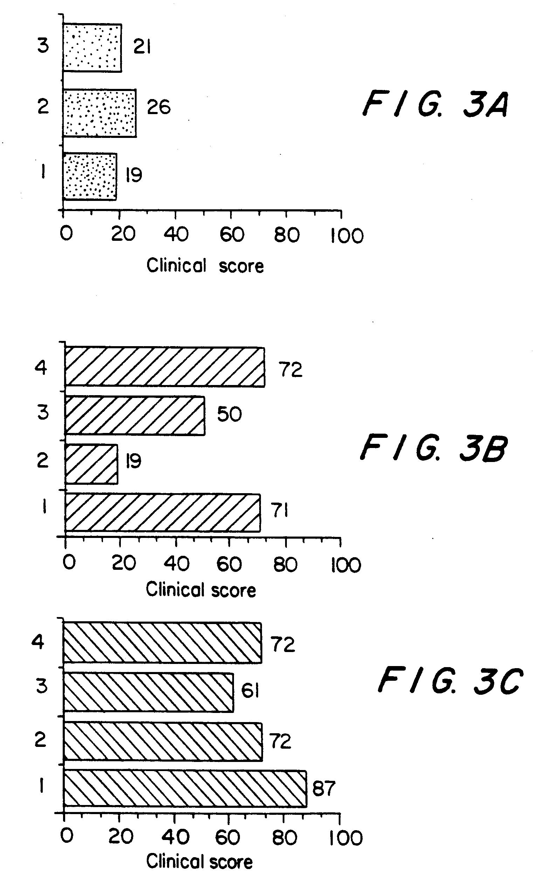

Animals were fed 10 mg of a bovine myelin preparation (Biopure, Boston, MA) with and without 150 micrograms of Lipid A as in Example 2 above, 3 times a week following the first attack of EAE on day 32 post-immunization. The animals were evaluated every other day for incidence of disease in a blinded fashion for 120 days. The disease was characterized by hind limb paralysis and incontinence. The disease was graded according to the clinical severity as follows: 0 = no abnormality; 1 = weakness, limpness, decreased activity; 2 = mild paraparesis; 3 = moderate paraparesis; 4 = severe paraparesis; 5 = death. The clinical severity score was generated by taking the actual score times the numbers of observations divided by the total potential maximal at-risk score (the maximal potential at-risk score assumes that every animal is a 4 in clinical severity) and multiplying times 100. The results are shown in Figure 3.

Figure 3C shows the control animals. The clinical scores for these animals (in Figures 3A-3C, each bar graph represents the results of one individual animal) ranged between 61 and 87 (Figure 3C, average 73). Oral bovine myelin administration reduced the clinical scores to 19-72 (Figure 3B, average 53) whereas oral administration of bovine myelin plus Lipid A further reduced the clinical scores to 19-26 (Figure 3A, average 22), demonstrating the efficacy of orally administering synergists with AD-suppressive agents to treat an ongoing autoimmune disease.

### EXAMPLE 7: Gut Epithelial Cells from Animals fed MBP plus LPS Express Differential Amounts of Class I and Class II Molecules

In these experiments gut epithelial cells were isolated from 6- to 8-week old female Lewis rats that had been fed either MBP or MBP plus LPS. Gut mucosal epithelial cells were obtained from normal Lewis rats, as described in Bland, P.W., et al. Immunol., 58:1, 1986. The small intestine, comprising duodenum and proximal jejunum was opened longitudinally, washed extensively in HBSS (Hanks balanced salt solution) and incubated at room temperature for 5 minutes in HBSS containing 0.5 mM EDTA. The villous epithelium and mucus were removed by rubbing the mucosa with a rubber policeman. The resulting clumps were then disrupted into a single cell suspension using a Pasteur pipette. Cells were then washed in RPMI 1640 medium containing 10% FCS (fetal calf serum) and, counted in a hemocytometer. Cells were then stained with monoclonal antibodies to Class I or Class II determinants and enumerated on a Fluoresence Activated Cell Sorter (FACS) as described in Santos, L.M. B. et al., Cellular Immunol (In press).

The amounts fed were 1 microgram of MBP or 1 microgram of MBP together with 1 microgram of LPS. The following percentages of cells expressed Class II-antigen: 53.8% of epithelial cells from control rats; 65.9% from MBP fed rat; and 16.9% from MBP plus LPS fed rats. The following percentages of cells expressed Class I-antigen: control rats 86.7%; MBP-fed rats 98.4%; MBP plus LPS fed rats 61%. These results demonstrate differential effects on Class II histocompatibility antigen expression of epithelial cells from MBP plus LPS fed rats. These results suggest that one of the possible mechanisms by which LPS acts involves differentially decreasing Class II expression (relative to Class I expression) on epithelial cells. Thus, these cells do not preferentially present antigen to CD4+ cells which are inducer (or helper) cells as opposed to suppressor cells.

### EXAMPLE 8:

In order to examine the mechanism of action of the synergists of the present invention, Lewis rats (5 per group) in which EAE had be induced as in Example 2, above were treated orally with 1 mg of GP-MBP alone, GP-MBP (1 mg) plus LPS (1 mg) (oral), IL-1 (200 micrograms, intraperitoneally administered) or fed GP-MBP (1 mg) + injected with IL-1 (200 micrograms intraperitoneally administered) 2-3 days after last feeding and the disease index measured. The results are set forth in Table 5 below:

**TABLE 5**

| Treatment | Disease Index |
|---|---|
| None (Control) | 23 |
| GP-MBP | 19 |
| GP-MBP + LPS | 17 |
| IL-1 | 28 |
| GP-MBP + IL-1 | 4 |

The results presented in Table 5 above show that oral administration of 1 milligram of GP-MBP reduced the Disease Index from 23 to 19 and that inclusion of LPS with the GP-MBP further lowered the Disease Index to 17. Injection of 200 micrograms of IL-1 alone (intrapertoneally) increased the Disease Index in the treated rats to 28. However, oral administration of 1 milligram of GP-MBP plus injection of 200 micrograms of IL-1 reduced the Disease Index to 4.

Therefore, the above results show that parenterally-administered IL-1 was able to synergize with oral MBP administration.

## Claims (Claims for the following Contracting State(s): AT, BE, CH, LI, DE, DK, FR, GB, IT, LU, NL, SE)

1. A pharmaceutical formulation for treating or preventing the clinical symptoms of a T-cell mediated or T-cell dependent autoimmune disease (AD) comprising an effective amount of
(a) an AD-suppressive agent in an oral or enteral dosage form which is selected from the group consisting of (i) autoantigens specific for said autoimmune disease, (ii) AD-suppressive fragments of said autoantigens, and (iii) AD-suppressive analogues of said autoantigens or fragments;
(b) at least one compound having the property of enhancing the AD-suppressive activity of said agent, wherein said compound administered in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

2. The pharmaceutical formulation of claim 1 wherein said autoantigen is myelin basic protein.

3. The pharmaceutical formulation of claim 1 wherein said compound is a bacterial lipopolysaccharide.

4. The pharmaceutical formulation of claim 1 wherein said compound is Lipid A.

5. The pharmaceutical formulation of claim 1 wherein said compound is suitable for oral or enteral administration.

6. The pharmaceutical formulation of claim 5 being suitable for administering said agent and said compound simultaneously.

7. The pharmaceutical formulation of claim 1 containing an amount of said agent individually effective to suppress or prevent the clinical symptoms of said disease.

8. The pharmaceutical formulation of claim 1 containing an amount of said agent and an amount of said compound said amounts being effective in combination to suppress said symptoms.

9. An oral or enteral pharmaceutical formulation comprising an effective amount of
(a) an AD-suppressive agent selected from the group consisting of (i) autoantigens specific for said autoimmune diseases, (ii) AD-suppressive fragments of said autoantigens, and (iii) AD-suppressive analogues of said autoantigens or fragments;
(b) at least one compound having the property of enhancing the AD-suppressive activity of said agent, wherein said compound, used in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

10. Use of (a) an AD-suppressive agent selected from (i) autoantigens specific for T-cell mediated or T-cell dependent immune diseases, (ii) AD-suppressive fragments of said autoantigens and (iii) AD-suppressive analogues of said autoantigens or fragments; (b) at least one compound having the property of enhancing the AD-suppressive activity of said agent, wherein said compound used in combination with said AD-suppressive agent imparts to said formulation one or both of the following properties:
(i) the property of inducing production and/or secretion of interleukin-1, and
(ii) the property of decreasing expression of class II histocompatibility molecules by gut epithelial cells; and (c) a physiologically acceptable carrier or diluent for preparing a pharmaceutical formulation for oral or enteral administration for treating or preventing the clinical symptoms of said T-cell mediated or T-cell dependent autoimmune diseases.

11. Use of at least one compound having the property of enhancing the AD-suppressive activity of an orally or enterally administered AD-suppressive agent selected from the group consisting of (i) autoantigens specific for a T-cell mediated or T-cell dependent autoimmune disease, (ii) AD-suppressive fragments of said autoantigens and (iii) AD-suppressive analogues of said autoantigens or fragments for preparing a medicament for treating or preventing the clinical symptoms of a T-cell mediated or T-cell dependent autoimmune disease, wherein said compound when used in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells.

12. The use according to claim 11, wherein said medicament is an oral or enteral medicament.

## Claims (Claims for the following Contracting State(s): ES)

1. A process for production of a pharmaceutical formulation for treating or preventing the clinical symptoms of a T-cell mediated or T-cell dependent autoimmune disease (AD) comprising an effective amount of
(a) an AD-suppressive agent in an oral or enteral dosage form which is selected from the group consisting of (i) autoantigens specific for said autoimmune disease, (ii) AD-suppressive fragments of said autoantigens, and (iii) AD-suppressive analogues of said autoantigens or fragments;
(b) at least one compound having the property of enhancing the AD-suppressive activity of said agent, wherein said compound administered in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

2. The process of claim 1 wherein said autoantigen is myelin basic protein.

3. The process of claim 1 wherein said compound is a bacterial lipopolysaccharide.

4. The process of claim 1 wherein said compound is Lipid A.

5. The process of claim 1 wherein said compound is suitable for oral or enteral administration.

6. The process of claim 5 being suitable for administering said agent and said compound simultaneously.

7. The process of claim 1 containing an amount of said agent individually effective to suppress or prevent the clinical symptoms of said disease.

8. The process of claim 1 containing an amount of said agent and an amount of said compound said amounts being effective in combination to suppress said symptoms.

9. A process for production of an oral or enteral pharmaceutical formulation comprising an effective amount of
(a) an AD-suppressive agent selected from the group consisting of (i) autoantigens specific for said autoimmune diseases, (ii) AD-suppressive fragments of said autoantigens, and (iii) AD-suppressive analogues of said autoantigens or fragments;
(b) at least one compound having the property of enhancing the AD-suppressive activity of said agent, wherein said compound, used in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

## Claims (Claims for the following Contracting State(s): GR)

1. A process for production of a pharmaceutical formulation for treating or preventing the clinical symptoms of a T-cell mediated or T-cell dependent autoimmune disease (AD) comprising an effective amount of
(a) an AD-suppressive agent in an oral or enteral dosage form which is selected from the group consisting of (i) autoantigens specific for said autoimmune disease, (ii) AD-suppressive fragments of said autoantigens, and (iii) AD-suppressive analogues of said autoantigens or fragments;
(b) at least one compound having the property of enhancing the AD-suppressive activity of said agent, wherein said compound administered in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

2. The process of claim 1 wherein said autoantigen is myelin basic protein.

3. The process of claim 1 wherein said compound is a bacterial lipopolysaccharide.

4. The process of claim 1 wherein said compound is Lipid A.

5. The process of claim 1 wherein said compound is suitable for oral or enteral administration.

6. The process of claim 5 being suitable for administering said agent and said compound simultaneously.

7. The process of claim 1 containing an amount of said agent individually effective to suppress or prevent the clinical symptoms of said disease.

8. The process of claim 1 containing an amount of said agent and an amount of said compound said amounts being effective in combination to suppress said symptoms.

9. A process for production of an oral or enteral pharmaceutical formulation comprising an effective amount of
(a) an AD-suppressive agent selected from the group consisting of (i) autoantigens specific for said autoimmune diseases, (ii) AD-suppressive fragments of said autoantigens, and (iii) AD-suppressive analogues of said autoantigens or fragments;
(b) at least one compound having the property of enhancing the AD-suppressive activity of said agent, wherein said compound, used in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells; and
(c) a physiologically acceptable carrier or diluent, the formulation being a combined preparation for simultaneous, separate or sequential administration of agent (a) and compound (b).

10. Use of (a) an AD-suppressive agent selected from (i) autoantigens specific for T-cell mediated or T-cell dependent immune diseases, (ii) AD-suppressive fragments of said autoantigens and (iii) AD-suppressive analogues of said autoantigens or fragments; (b) at least one compound having the property of enhancing the AD-suppressive activity of said agent; and wherein said compound used in combination with said AD-suppressive agent imparts to said formulation one or both of the following properties:
(i) the property of inducing production and/or secretion of interleukin-1, and
(ii) the property of decreasing expression of class II histocompatibility molecules by gut epithelial cells; and (c) a physiologically acceptable carrier or diluent for preparing a pharmaceutical formulation for oral or enteral administration for treating or preventing the clinical symptoms of said T-cell mediated or T-cell dependent autoimmune diseases.

11. Use of at least one compound having the property of enhancing the AD-suppressive activity of an orally or enterally administered AD-suppressive agent selected from the group consisting of (i) autoantigens sepcific for a T-cell mediated or T-cell dependent autoimmune disease, (ii) AD-suppresive fragments of said autoantigens and (iii) AD-suppressive analogues of said autoantigens or fragments for preparing a medicament for treating or preventing the clinical symptoms of a T-cell mediated or T-cell dependent autoimmune disease, wherein said compound when used in combination with said AD-suppressive agent, imparts to said formulation one or both of the following properties:
(i) the property of inducing production and/or secretion of interleukin-1, and
(ii) the property of decreasing expression of class II histocompatibility molecules by gut epithelial cells

12. The use according to claim 11, wherein said medicament is an oral or enteral medicament.

## Patentansprüche (Patentansprüche für folgende(n) Vertragsstaat(en): AT, BE, CH, LI, DE, DK, FR, GB, IT, LU, NL, SE)

1. Pharmazeutische Formulierung zur Behandlung oder Verhinderung von klinischen Symptomen einer T-Zell-vermittelten oder T-Zell-abhängigen Autoimmunkrankheit (AD), umfassend eine wirksame Menge von
(a) einem AD-suppressiven Mittel in einer oralen oder enteralen Dosierungsform, das ausgewählt ist aus der Gruppe, bestehend (i) Autoantigenen, die spezifisch für diese Autoimmunkrankheit sind, (ii) AD-suppressiven Fragmenten der Autoantigene und (iii) AD-suppressiven Analogen der Autoantigene oder Fragmente;
(b) mindestens einer Verbindung, die die Eigenschaft einer Verstärkung der AD-suppressiven Aktivität des Mittels aufweist, wobei die Verbindung, wenn sie in Kombination mit dem AD-suppressiven Mittel verabreicht wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt.

2. Pharmazeutische Formulierung nach Anspruch 1, worin das Autoantigen ein basisches Myelinprotein ist.

3. Pharmazeutische Formulierung gemäß Anspruch 1, worin die Verbindung ein bakterielles Lipopolysaccharid ist.

4. Pharmazeutische Formulierung gemäß Anspruch 1, worin die Verbindung Lipid A ist.

5. Pharmazeutische Formulierung gemäß Anspruch 1, worin die Verbindung für die orale oder enterale Verabreichung geeignet ist.

6. Pharmazeutische Formulierung gemäß Anspruch 5, die geeignet ist zur gleichzeitigen Verabreichung des Mittels und der Verbindung.

7. Pharmazeutische Formulierung gemäß Anspruch 1, die eine Menge des Mittels enthält, die individuell wirksam ist, die klinischen Symptome der Krankheit zu unterdrücken oder zu verhindern.

8. Pharmazeutische Formulierung gemäß Anspruch 1, enthaltend eine Menge des Mittels und eine Menge der Verbindung, wobei die Mengen in Kombination wirksam sind, um die Symptome zu unterdrücken.

9. Eine orale oder enterale pharmazeutische Formulierung, umfassend eine effektive Menge von
(a) einem AD-suppressiven Mittel, ausgewählt aus der Gruppe, bestehend aus (i) Autoantigenen, die für die Autoimmunkrankheiten spezifisch sind, (ii) AD-suppressiven Fragmenten der Autoantigene und (iii) AD-suppressiven Analogen der Autoantigene oder Fragmente;
(b) mindestens einer Verbindung, die die Fähigkeit zur Verstärkung der AD-suppressiven Aktivität des Mittels aufweist, wobei die Verbindung, wenn sie in Kombination mit dem AD-suppressiven Mittel verwendet wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt.

10. Verwendung von (a) einem AD-suppressiven Mittel, ausgewählt aus (i) Autoantigenen, die für die T-Zell vermittelten oder T-Zell abhängigen Immunkrankheiten spezifisch sind, (ii) AD-suppressiven Fragmenten der Autoantigene und (iii) AD-suppressiven Analogen der Autoantigene oder Fragmente;
(b) mindestens einer Verbindung, die die Fähigkeit zur Verstärkung der AD-suppressiven Aktivität des Mittels aufweist, wobei die Verbindung, wenn sie in Kombination mit dem AD-suppressiven Mittel verwendet wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel,
für die Herstellung einer pharmazeutischen Formulierung für orale oder enterale Verabreichung zur Behandlung oder Verhinderung der klinischen Symptome der T-Zell-vermittelten oder T-Zell-abhängigen Autoimmunkrankheiten.

11. Verwendung von mindestens einer Verbindung, die die Fähigkeit zur Verstärkung der AD-suppressiven Aktivität eines oral oder enteral verabreichten AD-suppressiven Mittels aufweist, ausgewählt aus der Gruppe, bestehend aus (i) Autoantigenen, die für die T-Zell-vermittelte oder T-Zell-abhängige Autoimmunkrankheit spezifisch sind, (ii) AD-suppressiven Fragmenten der Autoantigene und (iii) AD-suppressiven Analogen der Autoantigene oder Fragmente zur Herstellung eines Medikaments zur Behandlung oder Verhinderung von klinischen Symptomen einer T-Zell-vermittelten oder T-Zell abhängigen Autoimmunkrankheit, worin die Verbindung, wenn sie in Kombination mit dem AD-suppressiven Mittel verwendet wird, der Formulierung eine oder beiden der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen.

12. Verwendung gemäß Anspruch 11, worin das Medikament ein orales oder enterales Medikament ist.

## Patentansprüche (Patentansprüche für folgende(n) Vertragsstaat(en): ES)

1. Ein Verfahren zur Herstellung einer pharmazeutischen Formulierung zur Behandlung oder Verhinderung der klinischen Symptome einer T-Zell-vermittelten oder T-Zell-abhängigen Autoimmunkrankheit (AD), umfassend eine effektive Menge von
(a) einem AD-suppressiven Mittel in einer oralen oder enteralen Dosierungsform, das ausgewählt ist aus der Gruppe, bestehend (i) Autoantigenen, die spezifisch für diese Autoimmunkrankheit sind, (ii) AD-suppressiven Fragmenten der Autoantigene und (iii) AD-suppressiven Analogen der Autoantigene oder Fragmente;
(b) mindestens einer Verbindung, die die Eigenschaft einer Verstärkung der AD-suppressiven Aktivität des Mittels aufweist, wobei die Verbindung, wenn sie in Kombination mit dem AD-suppressiven Mittel verabreicht wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt, umfassend die im Stand der-Technik wohlbekannten Schritte.

2. Verfahren gemäß Anspruch 1, worin das Autoantigen basisches Myelinprotein ist.

3. Verfahren gemäß Anspruch 1, worin die Verbindung ein bakterielles Lipopolysaccharid ist.

4. Verfahren gemäß Anspruch 1, worin die Verbindung Lipid A ist.

5. Verfahren gemäß Anspruch 1, worin die Verbindung für die orale oder enterale Verabreichung geeignet ist.

6. Verfahren gemäß Anspruch 5, das geeignet ist zur gleichzeitigen Verabreichung des Mittels und der Verbindung.

7. Verfahren gemäß Anspruch 1, das eine Menge des Mittels enthält, die individuell wirksam ist, die klinischen Symptome der Krankheit zu unterdrücken oder zu verhindern.

8. Verfahren gemäß Anspruch 1, enthaltend eine Menge des Mittels und eine Menge der Verbindung, wobei die Mengen in Kombination wirksam sind, um die Symptome zu unterdrücken.

9. Verfahren zur Herstellung einer oralen oder enteralen pharmazeutischen Formulierung, umfassend eine wirksame Menge von
(a) einem AD-suppressiven Mittel, ausgewählt aus der Gruppe, bestehend aus (i) Autoantigenen, die für die Autoimmunkrankheiten spezifisch sind, (ii) AD-suppressiven Fragmenten der Autoantigene und (iii) AD-suppressiven Analogen der Autoantigene oder Fragmente;
(b) mindestens einer Verbindung, die die Fähigkeit zur Verstärkung der AD-suppressiven Aktivität des Mittels aufweist, wobei die Verbindung, wenn sie in Kombination mit dem AD-suppressiven Mittel verwendet wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt, umfassend die im Stand der Technik wohlbekannten Schritte.

## Patentansprüche (Patentansprüche für folgende(n) Vertragsstaat(en): GR)

1. Ein Verfahren zur Herstellung einer pharmazeutischen Formulierung zur Behandlung oder Verhinderung der klinischen Symptome einer T-Zell-vermittelten oder T-Zell-abhängigen Autoimmunkrankheit (AD), umfassend eine effektive Menge von
(a) einem AD-suppressiven Mittel in einer oralen oder enteralen Dosierungsform, das ausgewählt ist aus der Gruppe, bestehend (i) Autoantigenen, die spezifisch für diese Autoimmunkrankheit sind, (ii) AD-suppressiven Fragmenten der Autoantigene und (iii) AD-suppressiven Analogen der Autoantigene oder Fragmente;
(b) mindestens einer Verbindung, die die Eigenschaft einer Verstärkung der AD-suppressiven Aktivität des Mittels aufweist, wobei die Verbindung, wenn sie in Kombination mit dem AD-suppressiven Mittel verabreicht wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt, umfassend die im Stand der Technik wohlbekannten Schritte.

2. Verfahren gemäß Anspruch 1, worin das Autoantigen basisches Myelinprotein ist.

3. Verfahren gemäß Anspruch 1, worin die Verbindung ein bakterielles Lipopolysaccharid ist.

4. Verfahren gemäß Anspruch 1, worin die Verbindung Lipid A ist.

5. Verfahren gemäß Anspruch 1, worin die Verbindung für die orale oder enterale Verabreichung geeignet ist.

6. Verfahren gemäß Anspruch 5, das geeignet ist zur gleichzeitigen Verabreichung des Mittels und der Verbindung.

7. Verfahren gemäß Anspruch 1, das eine Menge des Mittels enthält, die individuell wirksam ist, die klinischen Symptome der Krankheit zu unterdrücken oder zu verhindern.

8. Verfahren gemäß Anspruch 1, enthaltend eine Menge des Mittels und eine Menge der Verbindung, wobei die Mengen in Kombination wirksam sind, um die Symptome zu unterdrücken.

9. Ein Verfahren zur Herstellung einer oralen oder enteralen pharmazeutischen Formulierung, umfassend eine wirksame Menge von
(a) einem AD-suppressiven Mittel, ausgewählt aus der Gruppe, bestehend aus (i) Autoantigenen, die für die Autoimmunkrankheiten spezifisch sind, (ii) AD-suppressiven Fragmenten der Autoantigene und (iii) AD-suppressiven Analogen der Autoantigene oder Fragmente;
(b) mindestens einer Verbindung, die die Fähigkeit zur Verstärkung der AD-suppressiven Aktivität des Mittels aufweist, wobei die Verbindung, wenn sie in Kombinationen mit dem AD-suppressiven Mittel verwendet wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel, wobei die Formulierung eine kombinierte Präparation für die gleichzeitige, getrennte oder aufeinanderfolgende Verabreichung von Mittel (a) und Verbindung (b) darstellt, umfassend die im Stand der Technik wohlbekannten Schritte.

10. Verwendung von (a) einem AD-suppressiven Mittel, ausgewählt aus (i) Autoantigenen, die für T-Zell-vermittelte oder T-Zell-abhängige Immunkrankheiten spezifisch sind, (ii) AD-suppressiven Fragmenten der Autoantigene und (iii) AD-suppressiven Analogen der Autoantigene oder Fragmente;
(b) mindestens einer Verbindung, die die Fähigkeit zur Verstärkung der AD-suppressiven Aktivität des Mittels aufweist, wobei die Verbindung, wenn sie in Kombination mit dem AD-suppressiven Mittel verwendet wird, der Formulierung eine oder beide der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen; und
(c) einem physiologisch akzeptablen Träger oder Verdünnungsmittel,
für die Herstellung einer pharmazeutischen Formulierung für orale oder enterale Verabreichung zur Behandlung oder Verhinderung der klinischen Symptome der T-Zell-vermittelten oder T-Zell-abhängigen Autoimmunkrankheiten.

11. Verwendung von mindestens einer Verbindung, die die Fähigkeit zur Verstärkung der AD-suppressiven Aktivität eines oral oder enteral verabreichten AD-suppressiven Mittels aufweist, ausgewählt aus der Gruppe, bestehend aus (i) Autoantigenen, die für die T-Zell-vermittelte oder T-Zell-abhängige Autoimmunkrankheit spezifisch sind, (ii) AD-suppressiven Fragmenten der Autoantigene und (iii) AD-suppressiven Analogen der Autoantigene oder Fragmente zur Herstellung eines Medikaments zur Behandlung oder Verhinderung von klinischen Symptomen einer T-Zell-vermittelten oder T-Zell abhängigen Autoimmunkrankheit, worin die Verbindung, wenn sie in Kombination mit dem AD-suppressiven Mittel verwendet wird, der Formulierung eine oder beiden der folgenden Eigenschaften verleiht:
(i) die Eigenschaft der Induktion einer Produktion und/oder Sekretion von Interleukin-1 und
(ii) die Eigenschaft einer Verminderung der Expression von Klasse II Histokompatibilitätsmolekülen durch Darmepithel-Zellen.

12. Verwendung gemäß Anspruch 11, worin das Medikament ein orales oder enterales Medikament ist.

## Revendications (Revendications pour l'(les) Etat(s) contractant(s) suivant(s): AT, BE, CH, LI, DE, DK, FR, GB, IT, LU, NL, SE)

1. Formulation pharmaceutique pour traiter ou prévenir les symptômes cliniques d'une maladie auto-immune dépendante des cellules T ou impliquant des cellules T (AD) comprenant une quantité efficace:
(a) d'un agent suppressif de AD sous une forme correspondant à un dosage oral ou entéral, qui est choisi dans le groupe formé par (i) les auto-antigènes spécifiques pour ladite maladie auto-immune, (ii) les fragments suppressifs de AD desdits auto-antigènes, et (iii) les analogues suppressifs de AD desdits antigènes ou fragments ;
(b) d'au moins un composé ayant la propriété de favoriser l'activité suppressive de AD dudit agent,
dans laquelle ledit composé administré en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable,
la formulation étant une préparation combinée destinée à une administration simultanée, séparée ou séquentielle de l'agent (a) et du composé (b).

2. Formulation pharmaceutique selon la revendication 1, dans laquelle ledit auto-antigène est la protéine basique de myéline.

3. Formulation pharmaceutique selon la revendication 1, dans laquelle ledit composé est un lipopolysaccharide bactérien.

4. Formulation pharmaceutique selon la revendication 1, dans laquelle ledit composé est le lipide A.

5. Formulation pharmaceutique selon la revendication 1, dans laquelle ledit composé est approprié pour une administration orale ou entérale.

6. Formulation pharmaceutique selon la revendication 5 qui est appropriée pour administrer ledit agent et ledit composé simultanément.

7. Formulation pharmaceutique selon la revendication 1, contenant une quantité dudit agent individuellement efficace pour supprimer ou prévenir les symptômes cliniques de ladite maladie.

8. Formulation pharmaceutique selon la revendication 1, contenant une quantité dudit agent et une quantité dudit composé, lesdites quantités étant efficaces en combinaison pour supprimer lesdits symptômes.

9. Formulation pharmaceutique orale ou entérale comprenant une quantité efficace:
(a) d'un agent suppressif de AD choisi dans le groupe formé par (i) les auto-antigènes spécifiques pour lesdites maladies auto-immunes, (ii) les fragments suppressifs de AD desdits auto-antigènes, et (iii) les analogues suppressifs de AD desdits antigènes ou fragments;
(b) d'au moins un composé ayant la propriété de favoriser l'activité suppressive de AD dudit agent, dans lequel ledit composé, utilisé en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable, la formulation étant une préparation combinée destirée à une administration simultanée, séparée ou séquentielle de l'agent (a) et du composé (b).

10. Utilisation (a) d'un agent suppressif de AD choisi dans le groupe formé par (i) les auto-antigènes spécifiques pour les maladies auto-immunes dépendant des cellules T ou impliquant des cellules T, (ii) les fragments suppressifs de AD desdits auto-antigènes, et (iii) les analogues suppressifs de AD desdits auto-antigènes ou fragments; (b) d'au moins un composé ayant la propriété de favoriser l'activité suppressive de AD dudit agent,
dans laquelle ledit composé utilisé en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable pour préparer une formulation pharmaceutique destinée à une administration orale ou entérale en vue de traiter ou de prévenir les symptômes cliniques desdites maladies auto-immunes dépendant des cellules T ou impliquant des cellules T.

11. Utilisation d'au moins un composé ayant la propriété de favoriser l'activité suppressive de AD d'un agent suppressif de AD administré par voie orale ou entérale choisi dans le groupe formé par (i) les auto-antigènes spécifiques pour ladite maladie auto-immune dépendant des cellules T ou impliquant des cellules T, (ii) les fragments suppressifs de AD desdits auto-antigènes, et (iii) les analogues suppressifs de AD desdits auto-antigènes ou fragments pour préparer un médicament en vue de traiter ou de prévenir les symptômes cliniques d'une maladie auto-immune dépendant des cellules T ou impliquant des cellules T, dans laquelle ledit composé quand on l'utilise en combinaison avec ledit agent suppressif de AD, impartit à la formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin.

12. Utilisation selon la revendication 11, dans laquelle ledit médicament est un médicament oral ou entéral.

## Revendications (Revendications pour l'(les) Etat(s) contractant(s) suivant(s): ES)

1. Procédé pour la production d'une formulation pharmaceutique pour traiter ou prévenir les symptômes cliniques d'une maladie auto-immune dépendante des cellules T ou impliquant des cellules T (AD) comprenant une quantité efficace:
(a) d'un agent suppressif de AD sous une forme correspondant à un dosage oral ou entéral, qui est choisi dans le groupe formé par (i) les auto-antigènes spécifiques pour ladite maladie auto-immune, (ii) les fragments suppressifs de AD desdits auto-antigènes, et (iii) les analogues suppressifs de AD desdits antigènes ou fragments ;
(b) d'au moins un composé ayant la propriété de favoriser l'activité suppressive de AD dudit agent,
dans laquelle ledit composé administré en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable,
la formulation étant une préparation combinée destinée à une administration simultanée, séparée ou séquentielle de l'agent (a) et du composé (b)comprenant les étapes contenues dans l'état de technique.

2. Procédé selon la revendication 1, dans lequel ledit auto-antigène est la protéine basique de myéline.

3. Procédé selon la revendication 1, dans lequel ledit composé est un lipopolysaccharide bactérien.

4. Procédé selon la revendication 1, dans lequel ledit composé est le lipide A.

5. Procédé selon la revendication 1, dans lequel ledit composé est approprié pour une administration orale ou entérale.

6. Procédé selon la revendication 5 qui est approprié pour administrer ledit agent et ledit composé simultanément.

7. Procédé selon la revendication 1, contenant une quantité dudit agent individuellement efficace pour supprimer ou prévenir les symptômes cliniques de ladite maladie.

8. Procédé selon la revendication 1, contenant une quantité dudit agent et une quantité dudit composé, lesdites quantités étant efficaces en combinaison pour supprimer lesdits symptômes.

9. Procédé pour la production d'une formulation pharmaceutique orale ou entérale comprenant une quantité efficace:
(a) d'un agent suppressif de AD choisi dans le groupe formé par (i) les auto-antigènes spécifiques pour lesdites maladies auto-immunes, (ii) les fragments suppressifs de AD desdits auto-antigènes, et (iii) les analogues suppressifs de AD desdits antigènes ou fragments ;
(b) d'au moins un composé ayant la propriété de favoriser l'activité suppressive de AD dudit agent, dans lequel ledit composé, utilisé en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable, la formulation étant une préparation combinée destinée à une administration simultanée, séparée ou séquentielle de l'agent (a) et du composé (b) comprenant les étapes contenues dans l'état de technique.

## Revendications (Revendications pour l'(les) Etat(s) contractant(s) suivant(s): GR)

1. Procédé pour la production d'une formulation pharmaceutique pour traiter ou prévenir les symptômes cliniques d'une maladie auto-immune dépendante des cellules T ou impliquant des cellules T (AD) comprenant une quantité efficace:
(a) d'un agent suppressif de AD sous une forme correspondant à un dosage oral ou entéral, qui est choisi dans le groupe formé par (i) les auto-antigènes spécifiques pour ladite maladie auto-immune, (ii) les fragments suppressifs de AD desdits auto-antigènes, et (iii) les analogues suppressifs de AD desdits antigènes ou fragments ;
(b) d'au moins un composé ayant la propriété de favoriser l'activité suppressive de AD dudit agent,
dans laquelle ledit composé administré en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable,
la formulation étant une préparation combinée destinée à une administration simultanée, séparée ou séquentielle de l'agent (a) et du composé (b) comprenant les étapes contenues dans l'état de technique.

2. Procédé selon la revendication 1, dans lequel ledit auto-antigène est la protéine basique de myéline.

3. Procédé selon la revendication 1, dans lequel ledit composé est un lipopolysaccharide bactérien.

4. Procédé selon la revendication 1, dans lequel ledit composé est le lipide A.

5. Procédé selon la revendication 1, dans lequel ledit composé est approprié pour une administration orale ou entérale.

6. Procédé selon la revendication 5 qui est approprié pour administrer ledit agent et ledit composé simultanément.

7. Procédé selon la revendication 1, contenant une quantité dudit agent individuellement efficace pour supprimer ou prévenir les symptômes cliniques de ladite maladie.

8. Procédé selon la revendication 1, contenant une quantité dudit agent et une quantité dudit composé, lesdites quantités étant efficaces en combinaison pour supprimer lesdits symptômes.

9. Procédé pour la production d'une formulation pharmaceutique orale ou entérale comprenant une quantité efficace:
(a) d'un agent suppressif de AD choisi dans le groupe formé par (i) les auto-antigènes spécifiques pour lesdites maladies auto-immunes, (ii) les fragments suppressifs de AD desdits auto-antigènes, et (iii) les analogues suppressifs de AD desdits antigènes ou fragments ;
(b) d'au moins un composé ayant la propriété de favoriser l'activité suppressive de AD dudit agent, dans lequel ledit composé, utilisé en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable, la formulation étant une préparation combinée destinée à une administration simultanée, séparée ou séquentielle de l'agent (a) et du composé (b) comprenant les étapes contenues dans l'état de technique.

10. Utilisation (a) d'un agent suppressif de AD choisi dans le groupe formé par (i) les auto-antigènes spécifiques pour les maladies auto-immunes dépendant des cellules T ou impliquant des cellules T, (ii) les fragments suppressifs de AD desdits auto-antigènes, et (iii) les analogues suppressifs de AD desdits auto-antigènes ou fragments; (b) d'au moins un composé ayant la propriété de favoriser l'activité suppressive de AD dudit agent,
dans laquelle ledit composé utilisé en combinaison avec ledit agent suppressif de AD, impartit à ladite formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin ; et
(c) d'un véhicule ou diluant physiologiquement acceptable pour préparer une formulation pharmaceutique destinée à une administration orale ou entérale en vue de traiter ou de prévenir les symptômes cliniques desdites maladies auto-immunes dépendant des cellules T ou impliquant des cellules T.

11. Utilisation d'au moins un composé ayant la propriété de favoriser l'activité suppressive de AD d'un agent suppressif de AD administré par voie orale ou entérale choisi dans le groupe formé par (i) les auto-antigènes spécifiques pour ladite maladie auto-immune dépendant des cellules T ou impliquant des cellules T, (ii) les fragments suppressifs de AD desdits auto-antigènes, et (iii) les analogues suppressifs de AD desdits auto-antigènes ou fragments pour préparer un médicament en vue de traiter ou de prévenir les symptômes cliniques d'une maladie auto-immune dépendant des cellules T ou impliquant des cellules T, dans laquelle ledit composé quand on l'utilise en combinaison avec ledit agent suppressif de AD, impartit à la formulation une ou les deux propriétés suivantes:
(i) la propriété d'induire la production et/ou la sécrétion d'interleukine-1, et
(ii) la propriété de diminuer l'expression des molécules d'histocompatibilité de classe II par des cellules épithéliales d'intestin.

12. Utilisation selon la revendication 11, dans laquelle ledit médicament est un médicament oral ou entéral.
